# EUROPEAN PATENT SPECIFICATION

(11) **EP 1 824 479 B1**
(45) Date of publication and mention of the grant of the patent: **05.11.2008**
(21) Application number: 05816322.1
(22) Date of filing: 06.12.2005
(51) Int. Cl.: A61K 31/423, C07D 263/58, A61P 25/00

(54) **PHENYLPIPERAZINE DERIVATIVES WITH A COMBINATION OF PARTIAL DOPAMINE-D2 RECEPTOR AGONISM AND SEROTONIN REUPTAKE INHIBITION**
PHENYLPIPERAZIN-DERIVATE MIT EINER KOMBINATION AUS TEILWEISEM DOPAMIN-D2-REZEPTOR-AGONISMUS UND SEROTONIN-WIEDERAUFNAHMEHEMMUNG
DERIVES DE PHENYLPIPERAZINE AVEC COMBINAISON D'AGONISME PARTIEL DE RECEPTEURS DE DOPAMINE-D2 ET INHIBITION DE REABSORPTION DE SEROTONINE

(30) Priority: 08.12.2004 EP 04106394; 08.12.2004 US 634074 P
(43) Date of publication of application: 29.08.2007
(73) Proprietor: Solvay Pharmaceuticals B.V., 1381 CP Weesp (NL)
(72) Inventor: FEENSTRA, Roelof W. Solvay Pharmaceuticals B.V., NL-1381 CP Weesp (NL); STOIT, Axel Solvay Pharmaceuticals B.V., NL-1381 CP Weesp (NL); TERPSTRA, Jan-Willem Solvay Pharmaceuticals B.V., NL-1381 CP Weesp (NL); PRAS-RAVES, Maria L. Solvay Pharmaceuticals B.V., NL-1381 CP Weesp (NL); MCCREARY, Andrew C. Solvay Pharmaceuticals B.V., NL-1381 CP Weesp (NL); VAN VLIET, Bernard, J. Solvay Pharmaceuticals B.V., NL-1381 CP Weesp (NL); HESSELINK, Mayke, B. Solvay Pharmaceuticals B.V., NL-1381 CP Weesp (NL); KRUSE, Cornelis, G. Solvay Pharmaceuticals B.V., NL-1381 CP Weesp (NL); VAN SCHARRENBURG, G.J.M. Solvay Pharmaceuticals BV, NL-1381 CP Weesp (NL)
(74) Representative: Verhage, Marinus
(86) International application number: PCT/EP2005/056507
(87) International publication number: WO 2006/061378

(56) References cited:
- EP-A- 0 900 792

## Description

The present invention relates to a group of novel and phenylpiperazine derivatives with a dual mode of action: serotonin reuptake in hibition and partial agonism on dopamine-D₂ receptors. The invention also relates to the use of a compound disclosed herein for the manufacture of a medicament giving a beneficial effect. A beneficial effect is disclosed herein or apparent to a person skil led in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the invention specific compounds disclosed herein a re used for the manufacture of a medicament useful in the treatment of disorders in which dopamine-D₂ receptors and serotonin reuptake sites are involved, or that can be treated via manipulation of those targets.

Compounds with a dual action as dopamine-D₂ antagonists and serotonin reuptake inhibitors are known from WO 00/023441, WO 00/069424 and WO 01/014330. This combination of activities is useful for the treatment of schizophrenia and other psychotic disorders: it enables a more complete treatment of a II disease symptoms. Benzoxa-zolone derivatives bearing a terminal phenyl group are disclosed in EP 0 900 792 A1. Those compounds however, have a dual action as dopamine-D₂ antagonists and 5-HT_{1A} agonists, and are devoid of serotonin reuptake inhibition.

The goal of the present invention was to provide further compounds with a dual action as partial dopamine-D₂ agonists and serotonin reuptake inhibitors.

The invention relates to a group of novel compounds of the formula (1): 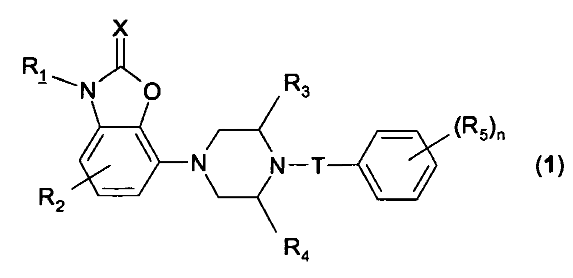 wherein: X = S or O,
R₁ is H, (C₁-C₆)alkyl, CF₃, CH₂CF₃, OH or O-(C₁-C₆)alkyl
   R₂ is H, (C₁-C₆)alkyl, halogen or cyano
   R₃ is H or (C₁-C₆)alkyl
   R₄ is H, (C₁-C₆)alkyl, optionally substituted with a halogen atom,
T is a saturated or unsaturated carbon cha in of 2-7 atoms, wherein one carbon atom may be replaced with a nitrogen atom, optionally substituted with an (C ₁-C₃)-alkyl, CF₃ or CH₂CF₃ group, an oxygen atom or a sulphur atom, which chain is optionally substituted with one or more substituents selected from the group consisting of (C₁-C₃)alkyl, (C₁-C₃)alkoxy, halogen, cyano, trifluoromethyl, OCF₃, SCF₃, OCHF₂ and nitro,
R₅ is a substituent selected from the group consisting of (C₁-C₃)alkyl, (C₁-C₃)alkoxy, halogen, cyano, trifluoromethyl, OCF₃, SCF₃, OCHF₂ and nitro,
n has the value 0-5,
and tautomers, stereoisomers and N-oxides thereof, as well as pharmacologically acceptable salts, hydrates and solvates of said compounds of formula (1) and its tautomers, stereoisomers and N-oxides,
with the proviso that when, X=O, R₁ is hydrogen or methyl, R₂ is hydrogen and n =1, R₅ is not 4-fluoro.

In the description of the substituents the abbreviation 'alkyl(C ₁₋₃)' means 'methyl, ethyl, n-propyl or isopropyl'.

N-oxides of the compounds mentioned above are in the scope of the present invention. Tertiary amines may or may not give rise to N-oxide metabolites. The extend to what N-oxidation takes place varies from trace amounts to a near quantitative conversion. N-oxides may be more active than their corresponding tertiary amines or less active. Whilst N-oxides are easily reduced to their corresponding tertiary amines by chemical means, in the human body this happens to varying degrees. Some N-oxides undergo nearly quantitative reductive conversion to the corresponding tertiary amines, in other cases the conversion is a mere trace reaction or even completely absent. (M.H. Bickel: " The pharmacology and Biochemistry of N-oxides", Pharmaco-logical Reviews, 21(4), 325 - 355, 1969).

It has been found that the compounds according to the invention show high affinity for both the dopamine D₂ receptor and the serotonin reuptake site. The compounds show activity at dopamine D₂ receptors with varying degree of agonism. All of the compounds show activity as inhibitors of serotonin reuptake, as they potentiate 5 - HTP induced behaviour in mice (B.L. Jacobs., 'An animal behaviour model for studying central serotonergic synapses', Life Sci., 1976, 19(6), 777-785).

In contrast to the use of full dopamine-D₂ receptor agonists or antagonists, the use of partial dopamine-D₂ receptor agonists offers a dynamic medication that self-adjusts on a moment-to-moment basis to the endogenous state of the patient. Thus, it provides the desired flexible modulation of the dopamine system and avoidance of the many adverse effects caused either by treatment using full dopamine-D₂ receptor agonists like bromocriptine (hallucinations, nausea, vomiting, dyskinesia, orthostatic hypotension, somnolescence) or full dopamine-D₂ receptor antagonists like haloperidol (emotional blunting, dysphoria, tardive dyskinesia). Because of these many adverse effects, full agonists and antagonists have found only very limited use in the therapy of depressive and anxiety disorders. Partial dopamine-D₂ receptor agonists not only show a flexible modulation and a favourable side-effect profile, they also have a pronounced anxiolytic profile in relevant animal models (Drugs of the Future 2001, 26(2) : 128-132).

Partial dopamine-D₂ receptor agonists, according to the present invention, are compounds that - when tested in a concentration response range -achieve activation in the functional cAMP cell based assay (as described below). Partial dopamine-D₂ receptor agonists will act as an agonist in cases when the endogenous synaptic tone of dopamine is low, or in the the presence of a full dopamine-D₂ receptor antagonist, and will act as an antagonist in cases when the endogenous synaptic tone of dopamine is high, or in the presence of a full dopamine D₂ receptor agonist. Like full agonists, partial dopamine-D₂ receptor agonists in general are active in sensitized systems. They induce contralateral turning in rats with unilateral 6-hydroxy-dopamine (6-OHDA) lesions in the substantia nigra pars compacta. In MPTP-treated common marmosets they produce potent and long-lasting reversal of motor symptoms (Drugs of the Future 2001, 26(2): 128-132). In contrast to full agonists, however, partial dopamine-D₂ agonists are substantially less active in non-sensitized systems: they hardly reverse reserpine induced hypolocomotion in rats.

For the treatment of CNS disorders involving an overactive dopaminergic system a pharmaceutical preparation combining partial dopamine-D₂ receptor agonistic activity having low intrinsic functional activity with serotonin reuptake inhibitory activity is recommended. In case of a disorder involving dopamine insufficiency a pharmaceutical preparation combining partial dopamine-D₂ receptor agonistic activity with high intrinsic functional activity and serotonin reuptake activity according to the invention has considerable advantages.

Disorders characterized by dynamic fluctuations in dopamine neurotransmission like bipolar depression and addiction will profit in particular from the flexible adjustment of the dopamine system by the partial dopamine-D₂ receptor agonists in the pharmaceutical preparation. Combining this "dopaminergic neurotransmission stabilizing" activity with serotonin reuptake inhibitory activity will enhance antidepressive and anxiolytic efficacy. The compounds can be used for the treatment of affections or diseases of the central nervous system caused by disturbances in the dopaminergic and serotonergic systems, for example: aggression, anxiety disorders, autism, vertigo, depression, disturbances of cognition or memory, Parkinson's disease, and in particular schizophrenia and other psychotic disorders.

Pharmaceutically acceptable salts may be obtained using standard procedur es well known in the art, for example by mixing a compound of the present invention with a suitable acid, for instance an inorganic acid such as hydrochloric acid, or with an organic acid.

### PHARMACEUTICAL PREPARATIONS

The compounds of the invention can be brought into forms suitable for administration by means of usual processes using auxillary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are th e pharmaceutically customary liquid or solid fillers and extenders, solvents, emulsifiers, lubricants, flavorings, colorings and/or buffer substances. Frequently used auxillary substances which may be mentioned are magnesium carbonate, titanium dioxide, la ctose, mannitol and other sugars, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions which are important and novel embodiments of the invention because of the presence of the compounds, more parti cularly specific compounds disclosed herein. Types of pharmaceutical compositions that may be used include but are not limited to tablets, chewable tablets, capsules, solutions, parenteral solutions, suppositories, suspensions, and other types disclosed he rein or apparent to a person skilled in the art from the specification and general knowledge in the art. In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### PHARMACOLOGICAL METHODS

### In vitro affinity for dopamine-D₂ receptors

Affinity of the compounds for dopamine-D₂ receptor s was determined using the receptor binding assay described by I. Creese, R. Schneider and S.H. Snyder: "[3H]-Spiroperidol labels dopamine receptors in rat pituitary and brain", Eur.J.Pharmacol., 46, 377 - 381, 1977.

### In vitro affinity for serotonin reuptake sites

Affinity of the compounds for serotonin reuptake sites was determined using the receptor binding assay described by E. Habert et al.,: "Characterisation of [3H]-paroxetine binding to rat cortical membranes", Eur.J.Pharmacol., 118, 107-114, 1985.

### Inhibition of forskolin-induced [³H]-cAMP accumulation

The *in vitro* functional activity at dopamine-D₂ receptors, including the intrinsic activity (ε) of the compounds of the invention was measured by their ability to inhibit forskolin-induced [³H]-cAMP accumulation.

Human dopamine D₂,_{L} receptors were cloned in fibroblast cell line CHO -K1 cells and obtained from Dr. Grandy, Vollum Institute, Portland, Oregon, USA. CHO cells were grown in a Dulbecco's modified Eagle's medium (DMEM) culture medium, supplemented with 10% heat-inactivated fetal calf serum, 2 mM glutamine, 1 mM pyruvate, 5000 units/ml penicillin, 5000 µg/ml streptomycin and 200 µg/ml G-418 at 37 °C in 93% air/7% CO₂. For incubation with test compounds, confluent cultures grown in 24 wells plates were used. Each condition or substance was routinely tested in quadruplicate. Cells were loaded with 1 µCi [³H]-adenine in 0.5 ml medium/well. After 2 hours, cultures were washed with 0.5 ml PBS containing 1 mM of the phosphor-diesterase inhibitor isobutylmethylxanthine (IBMX) and incubated for 20 min with 0.5 ml PBS containing 1 mM IBMX and forskolin with or without te st compound. After aspiration the reaction was stopped with 1 ml trichloroacetic acid 5% (w/v). The [³H]-ATP and [³H]-cAMP formed in the cellular extract were assayed as described by Solomon Y, Landos C, Rodbell M, 1974, A highly selective adenylyl cyclase assay, Anal Biochem 58:541-548 and Weiss S, Sebben M, Bockaert JJ, 1985, Corticotropin-peptide regulation of intracellular cyclic AMP production in cortical neurons in primary culture, J Neurochem 45:869-874. 0.8 ml Extract was passed over Dowex (50WX-4 200-400 mesh) and aluminumoxide columns, eluted with water and 0.1 M imidazole (pH=7.5). Eluates were mixed with 7 ml Insta -gel and radioactivity was counted with a liquid scintillation counter. The conversion of [³H]-ATP into [³H]-cAMP was expressed as the ratio in percentage radioactivity in the cAMP fraction as compared to combined radioactivity in both cAMP and ATP fractions, and basal activity was subtracted to correct for spontaneous activity.

Test compounds were obtained as 10 mM stock solutions in 100% DMSO, and diluted in PBS/IBMX to final concentrations. Typically, compounds were used in concentrations that ranged from 10⁻¹⁰M to 10⁻⁵M. From quadruplicate data counts, the mean was taken as an estimate for drug-induced, receptor-mediated effects at specified second messenger accumulation, expressed as percentage of control values (forskolin-stimulated cAMP accumulation, subtracted by basal activity). By using the non-linear curve-fitting program INPLOT or the Excel-add-in XL-Fit, mean values were plotted against drug concentration (in molar) and a sigmoid curve (four-parameter logistic curve) was constructed. The maximal forskolin -induced stimulated conversion is taken as maximum value and the maximal inhibition (usually at drug concentrations 10⁻⁶ M or 10⁻⁵ M) as minimum and these values were fixed during the fitting process. Thus, concentrations of the compound, causing 50% of the maximally obtained inhibition of forskolin-induced cAMP accumulation (EC₅₀), are averaged over several experiments and presented as mean pEC₅₀ ± SEM. Antagonist potency is assessed by co-incubating cells with a fixed agonist concentration and specified antagonist concentrations. Curve fitting procedures are identical to those used for estimating EC₅₀ values. Thus IC₅₀ values, i.e. the concentration that is able to achieve 50% of maximal antagonism that can be achieved by this compound. IC₅₀ values are corrected using a Cheng-Prussoff equation, correcting it for agonist concentration and EC₅₀ values that is obtained in the same experiment. Thus, K_{b} = IC₅₀ / (1+ [agonist]/EC₅₀, agonist).The corresponding pA₂ value is -log (K_{b}). Concentration-response curve fitting allows estimation of pEC₅₀ values and of maximal achievable effect (intrinsic activity or efficacy (ε). A full receptor agonist has ε = 1, a full receptor antagonist has ε = 0, and a partial receptor agonist has an intermediate intrinsic activity.

### DOSAGES

The affinity of the compounds of the invention for dopamine-D₂ receptors and serotonine reuptake sites was determined as described above. From the binding affinity measured for a given compound of formula (1), one can estimate a theoretical lowest effective dose. At a concentration of the compound equal to twice the measured Kᵢ-value, 100% of the receptors likely will be occupied by the compound. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 - 1000 mg/kg, preferably 0.1-100 mg/kg of patient's bodyweight.

### TREATMENT

The term 'treatment' as used herein refers to any treatment of a mammalian, preferably human condition or disease, and includes: (1) preventing the disease or condition from occurring in a subject which may be predisposed to the disease but has not yet been diagnosed as having it, (2) inhibiting the disease or condition, i.e., arresting its development, (3) relieving the disease or condition, i.e., causing regression of the condition, or (4) relieving the conditions caused by the disease, i.e., stopping the symptoms of the disease.

The preparation of the compounds having formula (I) will now be described in more detail in the following Examples.

### EXAMPLES

The H-atom of the N-H moiety of the phenylpiperazine part of the compounds of formula (1), the 'amines' **I-H** to **IX-H** can be replaced by **Q** by the general method A (*see above*) leading to the compounds of the invention which are listed in table 1 (*see below*).

### Method A:

The compounds were prepared via the synthesis depicted in scheme A1: an amine was reacted with **Q-X** (X = leaving group like *e.g.* Cl, Br, I) in *e.g.* acetonitrile or butyronitrile with Et(*i*-Pr)₂N acting as a base, in some cases KI (or Nal) was added. Et₃N can be used instead of Et(*i*-Pr)₂N.

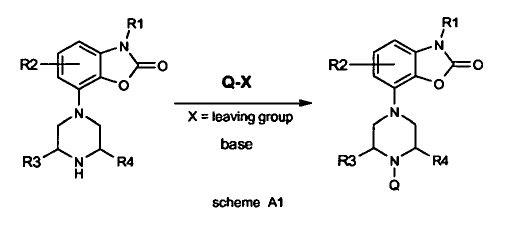

### Example 1:

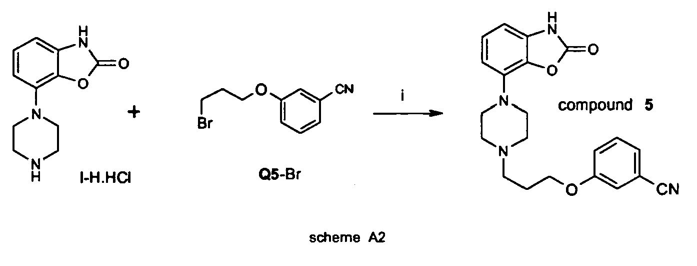

### Scheme A2, step i:

A suspension of the piperazine hydrochloride **I-H.**HCl (5.11 g, 20 mmol), 6.0 g (25 mmol) of the bromide, KI (7.75 g) and DIPEA (10,6 ml ; 60 mmol) in 75 ml acetonitril was heated on an oilbath at reflux under nitrogen for 20 hours. After cooling down to room temperature the suspension was filtered with suction and concentrated *in vacuo*. Purification by column chromatography on silica gel (eluent: DCM/MeOH/NH ₃ = 920/75/5) gave two fractions of compound **5** (both 2.9 g) which were together recrystallised from 75 ml of acetonitril, yielding 4.48 g of compound 5 as an off-white solid. M.p.: 145-9 °C.

**Table 1: examples of compounds of the invention.**

| | | | | | | |
|---|---|---|---|---|---|---|
| Structures of the phenylpiperazine part of the compounds of formula (1), herein termed 'amines', and groups 'Q' are given below. In the column 'method', the general method is given and the next column gives the leaving group. | | | | | | |

| **comp** | **amine** | **Group Q** | **meth.** | **L-group** | **salt** | **melting r. °C** |
|---|---|---|---|---|---|---|
| | | | | | | |
| 1 | I | 1 | A | Br | free base | 169-171 |
| 2 | I | 2 | A | Br | free base | 150-152 |
| 3 | I | 3 | A | Br | HCl | 238-240 |
| 4 | I | 4 | A | Br | free base | 139-142 |
| 5 | I | 5 | A | Br | free base | 145-149 |
| 6 | I | 6 | A | Br | free base | 165-168 |
| 7 | I | 7 | A | Br | FUM | 170-175 |
| 8 | I | 8 | A | Br | free base | 166-169 |
| 9 | I | 9 | A | Br | free base | 153-155 |
| 10 | I | 10 | A | Br | free base | 180-183 |
| 11 | I | 11 | A | Br | free base | 130-132 |
| 12 | I | 12 | A | I | free base | 178-179 |
| 13 | I | 13 | A | I | free base | 170-171 |
| 14 | I | 15 | A | I | HCl | 230-231 |
| 15 | I | 16 | A | I | HCl | 195-196 |
| 16 | I | 17 | A | I | HCl | 229-231 |
| 17 | I | 18 | A | Br | free base | 140-142 |
| 18 | I | 19 | A | Br | free base | 84-86 |
| 19 | I | 20 | A | I | free base | 166-168 |
| 20 | I | 21 | A | I | HCl | 168-170 |
| 21 | I | 22 | A | I | free base | 146-148 |
| 22 | I | 23 | A | I | HCl | 161-163 |
| 23 | I | 24 | A | Br | free base | 164-166 |
| 24 | I | 25 | A | Br | free base | 189-190 |
| 25 | I | 26 | A | Br | free base | 151-153 |
| 26 | II | 5 | A | Br | HCl | 251-254 |
| 27 | II | 6 | A | Br | HCl | 230-235 |
| 28 | II | 17 | A* | I | free base | 156-8 |
| 29 | V | 5 | A | Br | free base | 147-149 |
| 30 | V | 6 | A | Br | free base | 123-125 |
| 31 | V | 9 | A | Br | free base | 95-97 |
| 32 | V | 13 | A | I | free base | 152-154 |
| 33 | V | 14 | A | I | HCl | 174-176 |
| 34 | V | 16 | A | I | HCl | 216-218 |
| 35 | V | 17 | A* | I | free base | 87-9 |
| 36 | VI | 5 | A | Br | free base | 171-2 |
| 37 | VI | 6 | A | Br | HCl | 242-4.5 |

| | | | | | | |
|---|---|---|---|---|---|---|
| **): see scheme 12-17b* | | | | | | |

The piperazines used in these methods are indicated as **I-H** to **IX-H:**

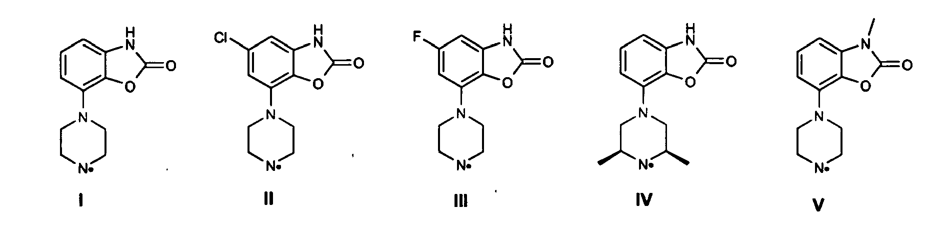

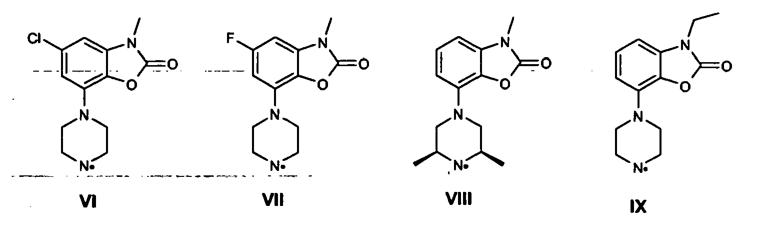

The syntheses of the piperazines **I**-H, **III**-H and **V**-H are described in WO97/36893.

### Synthesis of amine II-H:

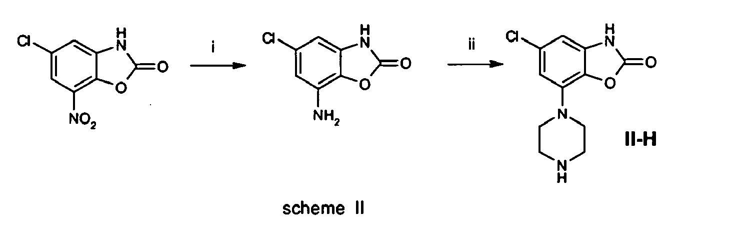

The synthesis of the starting material has been described (patent DE487014).

### Scheme II, step i:

30 g ((0.14 mol) of the starting material was suspended in 600 ml of MeOH. Then a small amount of Raney nickel was added after which hydrogenation was started (atmospheric, room temperature). After 24 hours 7.2 liters (theoretical amou nt 9.4 liters) of hydrogen was absorbed. To the reaction mixture 150 ml of THF was added and another small amount of Raney nickel. After one hour the reaction mixture was filtered over hyflo, the residu washed with THF. The filtrate was concentrated *in vacuo,* yielding 25.2 g (98%) of the correspondig aniline.

### Scheme II, step ii:

24.2 g (131.2 mmol) of the aniline of the previous step and 25.8 g (144.3 mmol) of bis (2-chloroethyl)amine were suspended in 675 ml of chlorobenzene. While stirring, 25 ml of solvent were distilled off with the aid of a Dean-Stark apparatus. After removal of the Dean-Stark apparatus, the reaction was allowed to reflux for 48 hours. When the reaction mixture had come to room temperature, the mixture was decanted and the residu washed twice with Et₂O. Then 400 ml of MeOH were added after which the mixture was warmed until almost all of the residu was dissolved. Then 200 ml of silica were added after which the whole was concentrated in vacuo. Then the residu was put on top of a flash chromatography column using DMA 0.75 as the eluent. After removal of the solvent a residu was isolated which was suspended in about 100 ml of acetonitrile and stirred for 4 hours. Filtration and drying yielded 17 g of the desired piperazine **II**-H as a free base.

### Synthesis of amine IV-H:

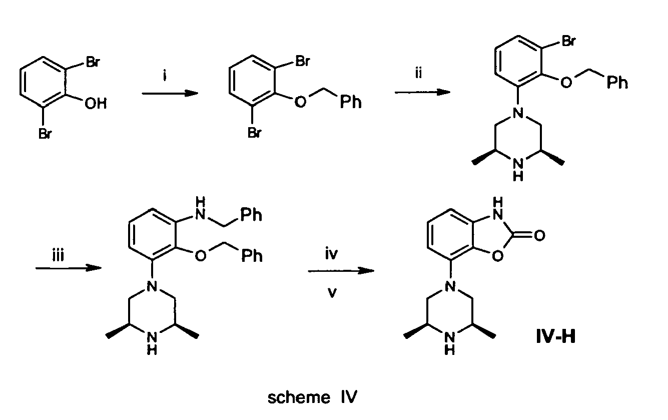

### Scheme IV, step i:

20.5 g (81.3 mmol) of dibromophenol and 20 g of potassium carbonate were suspended in 400 ml of aceton, after which 15.7 ml of benzylbromide were added. The reaction mixture was refluxed for 24 hours. Af ter the mixture had reached room temperature, it was concentrated in vacuo. Subsequently water was added and CH₂Cl₂. The organic layer was filtered with a water repellant filter, the dry filtrate concentrated in vacuo after which it was dissolved again in 200 ml of acetonitrile. Subsequently, 15 ml of piperidine were added after which the temperature was raised to 60 °C for one hour. The reaction mixture was concentrated in vacuo and CH₂Cl₂ was added. The latter was washed with: 1 N HCl (3x), water, 2N NaOH, and again water. The organic layer was filtered with a water repellant filter, the dry filtrate concentrated in vacuo yielding 27.6 g (99%) of the corresponding benzylated phenol.

### Scheme IV, step ii:

The toluene used in this experiment was degassed for three hours prior to usage. 1.48 g (1.61 mmol) of Pd₂(dba)₃ and 3.02 g (4.85 mmol) of BINAP were put into 400 ml of toluene after which the mixture was stirred and heated to 105 °C for 0.5 hours after which the mixture was allowed to cool to room temperature. Subsequently were added to the reaction mixture: 27.6 g (80.7 mmol) of the benzylated compound (step i) dissolved in 50 ml of toluene, 9.2 g (80.7 mmol) of the (α,α')-dimethylpiperazine and 10.08 g (104.9 mmol) of sodium *tert*.butoxide. The resulting mixture was heated at 105 °C for 20 hours, after which it was allowed to reach room temperature. The mixture was diluted with CH₂Cl₂ after which it was filtered over hyflo and concentrated in vacuo. The residu was put on top of a flash chromatography column (SiO₂) using DMA 0.125. The combined product containing fractions yielded after concentration in vacuo 7.7 g (26%) of the almost pure phenylpiperazine.

### Scheme IV, step iii:

This step was done analogously to the procedure described in the previous step ii (scheme IV). In this case benzylamine was used in the Buchwald reaction. Yield: 88%.

### Scheme IV, step iv:

7 ml (98 mmol) of acetyl chloride was added dropwise to 70 ml of cooled absolute ethanol, stirring was continued for 15 minutes. The latter solution was added to a solution of 11.5 g (28.7 mmol) of the dibenzyl product of step iii in 250 ml of methanol. Subsequently 1.5 g of Pd/C (10%) was added, after which the reaction mixture was hydrogenated for 24 hours. The mixture was filtered over hyflo, the filtrate concentrated in vacuo. The residu containing the amino phenol HCl salt was *directly* used in step v.

### Scheme IV, step v:

The residu (28.7 mmol) obtained in step iv, 52 ml of DIPEA (298 mmol), and 20.9 g (129 mmol) of CDI were added to 750 ml of THF after which the mixture was refluxed for 20 hours under a nitrogen atmosphere. After cooling to room temperature, the mixture was concentrated in vacuo, to the residu CH ₂Cl₂ and 5% NaHCO₃ were added, the whole being stirred for one hour. Extraction with CH ₂Cl₂ (3x), the water fraction was concentrated and extracted again (CH ₂Cl₂, 3x). The combined organic fractions were concentrated in vacuo, the residu contained a considerable amount of imidazol. The whole was solved in 120 ml of acetonitrile after which the solution was allowed to reach room temperature. The precipitate which formed was filtered yielding almost pure piperazine **IV.**

### Synthesis of amine V-H:

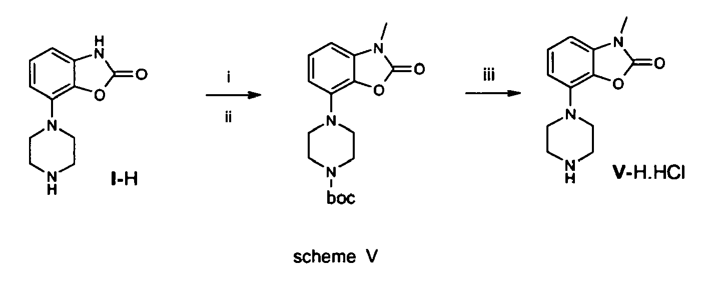

### Scheme V, steps i, ii and iii:

Synthesis of **V-H** has been described in WO97/36893. The steps i, ii a nd iii were done analogously to steps i, ii and iii in scheme VI.

### Synthesis of amine VI-H:

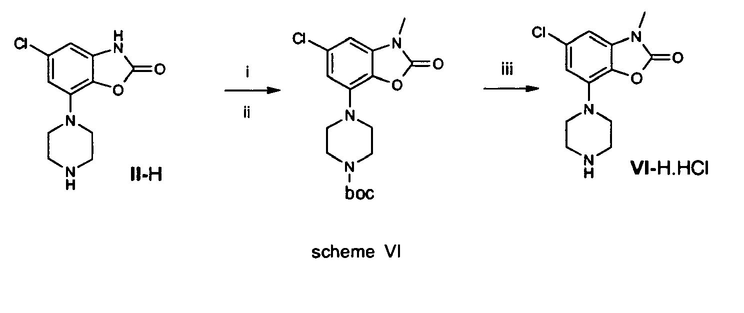

### Scheme VI, step i:

While stirring, 3.8 g (15 mmol) of piperazine **II-H** were suspended in 5.48 ml (31.5 mmol) of DIPEA and the mixture was brought to -40 °C. A solution of 3.14 g (14.4 mmol, 0.96 eq) of Boc-anhydride in 30 ml of CH₂Cl₂ was added dropwise in 100 minutes. Stirring was continued at -40 °C (1 hour), then at -30 °C (2 hours), and the reaction mixture was allowed to come to room temperature (16 hours). T hen water and some MeOH were added after which it was extracted with CH ₂Cl₂. The combined organic fractions were filtered with a water repellant filter, the dry filtrate mixed with 50 ml of silica after which the whole was concentrated in vacuo. Then the residu was put on top of a dry chromatography column (SiO₂) using CH₂Cl₂/MeOH (98/2) as the eluent. The part of the column containing the product was cut out, and the product washed out of the column material with CH ₂Cl₂/MeOH (98/2) yielding 3.55 g (67%) of the desired N-Boc **II.**

### Scheme VI, step ii:

4.5 g (12.7 mmol) N-Boc **II** together with 5.8 g (3.3 eq) of potassium carbonate were suspended in 100 ml of aceton. While stirring, the reaction mixture was cooled to - 10 °C after which 0.87 ml (14 mmol, 1.1 eq) of methyl iodide was added dropwise. After 15 minutes, the reaction mixture was allowed to reach room temperature and stirring was continued for 14 hours. Subsequently, the reaction mixture was concentrated in vacuo, the residu mixed with water and CH ₂Cl₂. The water layer was separated and extracted twice with CH₂Cl₂. The combined organic layers were filtered with a water repellant filter, the dry filtrate concentrated in vacuo yielding 4.5 g (98%) of the corresponding N'-methylated N-Boc **II.**

### Scheme VI, step iii:

While stirring at -10 °C, 5 ml of acetyl chloride (70.4 mmol, 5.8 eq) was added dropwise to 65 ml of ethanol. The latter solution was added to 4.5 g (12.2 mmol)of the N'-methylated N-Boc **II** isolated in step *ii.* The resulting mixture was stirred for 3 hours at 55 °C, then the reaction mixture was allowed to reach room temperature and stirring was continued for 14 hours. Subsequently, the mixture was concentrated in vacuo after which the residu was suspended in di -isopropyl ether and stirred for 2 hours. The precipitate was isolated by filtration yielding 3.6 g (97%) of piperazine **VI**-H.HCl.

### Synthesis of amine VII-H:

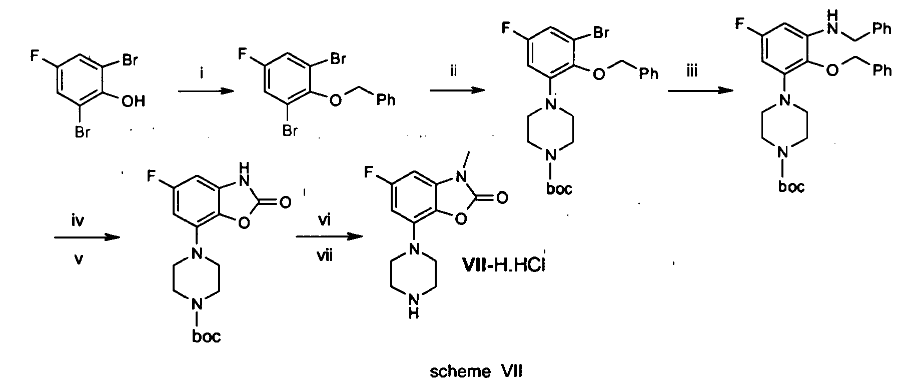

### Scheme VII, step i:

This step was done analogously to step i in scheme IV. After chromatograhic purification an oil containing th e benzylated product, was isolated in 88% yield. The oil solidified upon standing.

### Scheme VII, step ii:

This step was done analogously to step ii in scheme IV. Boc-piperazine was used in this Buchwald reaction. Yield after chromatographic purification: 4 4% of a brown oil.

### Scheme VII, step iii:

This step was done analogously to the procedure described in the previous step ii (scheme VII). In this case benzylamine was used in the Buchwald reaction. Yield after chromatographic purification: 73% of a brown o il.

### Scheme VII, step iv:

11.91 g (24.3 mmol) of the dibenzylated product isolated in previous step iii (scheme VII) was suspended in a mixture of 110 ml of ethanol, 72 ml of water and 11 ml of acetic acid. While stirring, 0.5 g of Pd(OH)₂/C was added and hydrogenation was started for 6 days. After one day and after 3 days an additional small amount of Pd(OH)₂/C was added. The reaction mixture was filtered over hyflo, the filtrate concentrated in vacuo. The residu was treated with toluene and concentrated in vacuo, this procedure was repeated, leaving a dark sirup 7.9 g (88%), containing the amino phenol.

### Scheme VII, step v:

This step (ring closure with CDI) was done analogously to step v in scheme IV. The crude product after work up was chromatographed (f lash column, SiO₂, eluent DCM/MeOH 97/3) yielding 7.6 g of an impure brown foam. A second chromatography (flash column, SiO₂, eluent EtOAc/petroleum ether 1/2) yielded 3.3 g (42%) of pure brown foam, containing the N-Boc protected benzoxazolinone piperazine.

### Scheme VII, step vi:

This methylation step was done analogously to the procedure described in step ii (scheme VI). Yield: 98% of a brown foam of 97% purity.

### Scheme VII, step vii:

This deprotection step was done analogously to the procedure described in step iii (scheme VI). Yield: 94% of a light pink solid of 98% purity, containing the product **VII**-H.HCl.

### Synthesis of amine VIII-H:

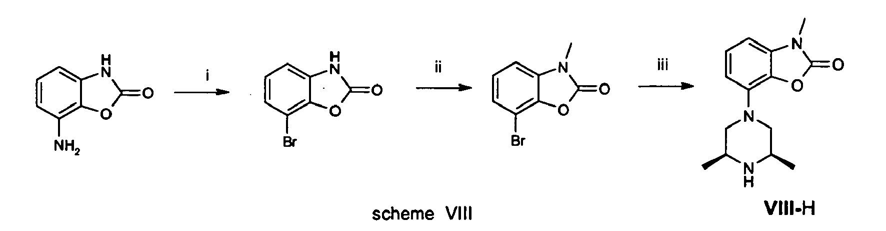

### Scheme VIII, step i:

The starting material synthesis has been described in EP0189612.
4.91 g (32.7 mmol) of the anilin was suspended in 75 ml of 48% of HBr/water, while it was cooled to -5 °C. Subsequently 2.27 g (33 mmol) of sodium nitrite dissolved in 4 ml of water, were added dropwise during 15 minutes. Stirring was continued at 0 oC for 15 minutes.

Subsequently, the reaction mixture was added, in one time, to a 0 °C solution of 2.42 g (16.9 mmol) CuBr in 20 ml of 48% HBr/water. After 30 minutes the reaction mixture was heated to 85 oC for one hour, after which it was allowed to reach room temperature, stirring was continued for 14 hours. To the mixture diethyl ether and water were added, after shaking the organic layer was isolated which was washed with water. The organic layer, together with some silica, was concentrated in vacuo, and the residu was put on top of a flash chromatography column (SiO₂) using Et₂O/petroleum ether (1/1), and later on pure Et₂O as the eluent. The combined product containing fractions yielded after concentration in vacuo 3.3 g (47%) of the desired corresponding bromo product.

### Scheme VIII, step ii:

This step was carried out identical to step *ii* in scheme VI. Yield: 92% of the corresponding methylated bromo compound.

### Scheme VIII, step iii:

In the following order 6.82 g (29.9 mmol) of the methylated bromo compound, 4.03 g (35.9 mmol) of the dimethyl piperazine, 13.6 g (41.9 mmol) of Cs₂CO₃, 1.42 g (2.99 mmol) of X-Phos (see Huang et al., J. Am. Chem. Soc.,125(2003)6653 ). and 0.55 g (0.6 mmol) of Pd₂(dba)₃ were added to 225 ml of toluene which was degassed for 4 hours prior to usage. While stirring and under a nitrogen atmosphere the temperature was raised to 100 °C for 20 hours, after which it was allowed to reach room temperature. The mixture was diluted with CH ₂Cl₂ after which it was filtered and concentrated in vacuo. The residu was put on top of a flash chromatography column (SiO₂) using DMA 0.25. The combined product containing fractions yielded after concentration in vacuo 0.73 g (9%) of the desired pure piperazine **VIII**-H.

### Synthesis of amine IX-H:

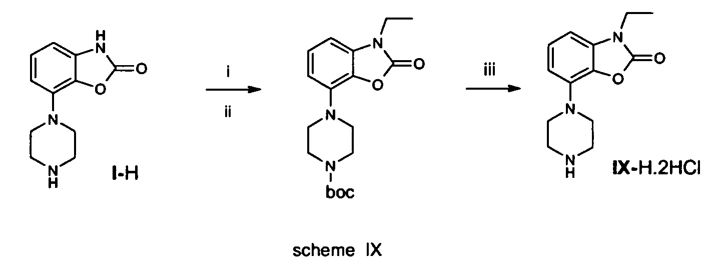

### Scheme IX, steps i, ii and iii:

Synthesis of **I**-H has been described in WO97/36893. The steps i, ii and iii were done analogously to steps i, ii and iii in scheme VI.

Below, the various forms **Q1-Q26** are given:

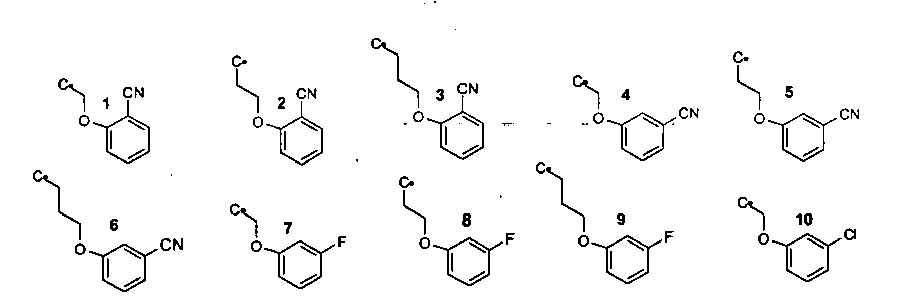

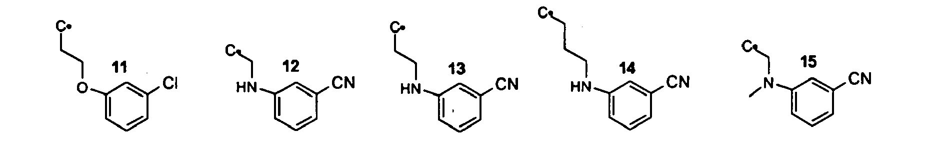

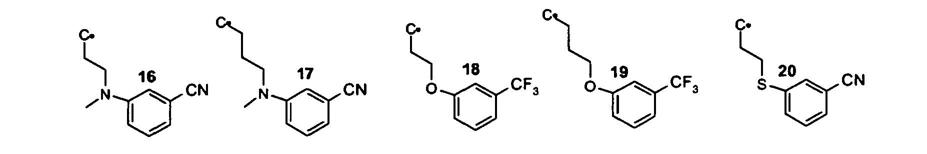

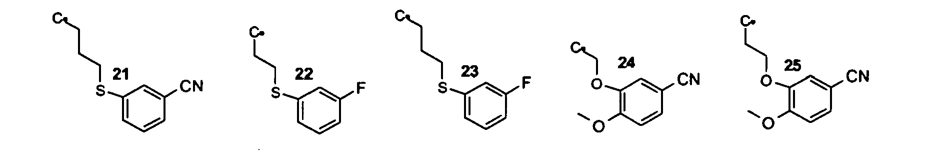

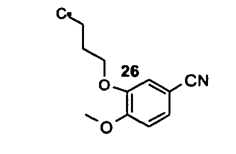

In these formulae 'Q', the dot represents the attachments to the phenylpipera zine part of the compounds of formula (1).

### Synthesis of Q1-11, 18-19, 24-26:

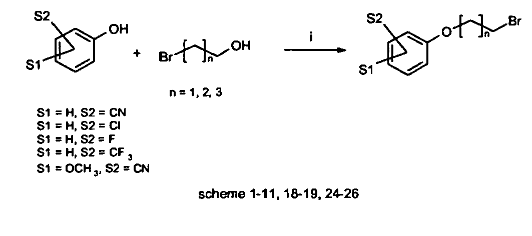

All starting phenols (except where S1= 2-OCH₃, S2= 5-CN, see scheme 1-11, 18-19, 24-26; b) and alcohols were commercially available.

### Scheme 1-11, 18-19, 24-26; step i:

To a stirred solution of 3-fluorophenol (7 ml, 77.43 mmol), PPh₃ (26.4 g, 101 mmol) and 4-bromo-1-butanol (13.35 ml, 123.9 mmol) in 275 ml Toluene at 0 °C under an nitrogen atmosphere, was added dropwise a solution of DIAD (30.46 ml, 155 mmol) in 60 ml toluene (the temperature was kept between 0 °C and 5 °C during the addition). The reaction mixture was stirred for 20 hours at room temperature. The solution was evaporated under reduced pressure and 300 ml of Et₂O/petroleum ether 1:1 was added to the residue. The precipitate which formed during stirring for 30 minutes at room temperature was filtered off and washed 2 times with Et₂O/petroleum ether 1:3 (50 ml). The filtrate was evaporated under reduced pressure and the residue was chromatographed using DCM/petro leum ether 1:4 as eluent to give 18.39 g (96%) of the ether **Q9**-Br as a colorless oil.

Synthesis of phenols with S1= 2-OCH₃ and S2= 5-CN:

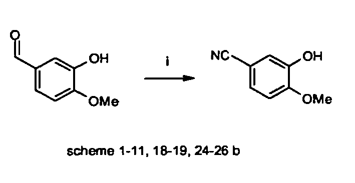

### Scheme 1-11, 18-19, 24-26; b, step i:

A mixture of 3-hydroxy-4-methoxybenzaldehyde (31.4 g, 206.6 mmol) and hydroxylamine monohydrochloride (18.7 g, 268.6 mmol) in 165 ml formic acid was refluxed for 1 h. The reaction mixture was cooled to room temperature and 1 litre of ice water was added. The precipitate was filtered and the residu washed with ice water. The solid was further dried by co-evaporation with acetonitrile yielding 13.84 g (45%) of a solid, containing the 2-methoxy-5-cyano-phenol.

### Synthesis of Q12-17:

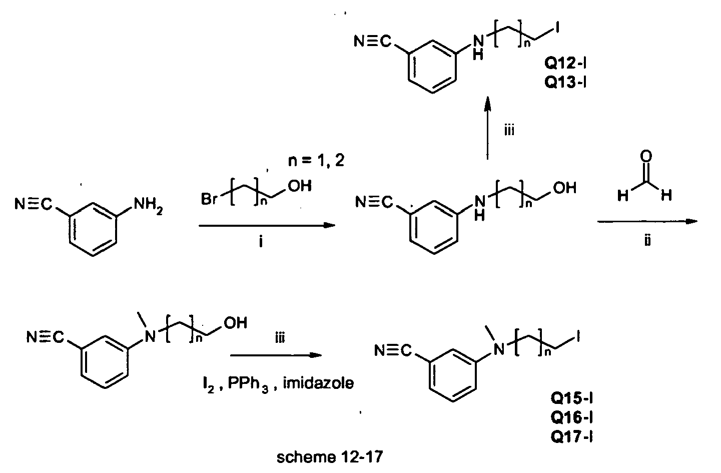

### Scheme 12-17 step i:

For n =2 and 3-cyanoaniline:

A mixture of 3-cyanoaniline (2.95 g, 25 mmol), Nal (7.5 g, 50 mmol), DIPEA (4.3 ml, 25 mmol) and 3-bromo-1-propanol (2.25 ml, 25 mmol) in 50 ml butyronitrile was refluxed for 24 h. The solvent was evaporated under reduced pressure. H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH/NH₃ 92/7.5/ 0.5 as the eluent to give 3.45 g (78.4%) of the N-alkylated product **Q13**-OH as an oil.

### Scheme 12-17 step ii:

To a stirred solution of the N-alkylated product from step i (3.3 g, 18.8 mmol), 37% formaldehyde solution (14 ml, 188 mmol) and NaCNBH₃ (3.7 g, 56.4 mmol) in 70 ml CH₃CN was added dropwise 1.8 ml glacial acetic acid in 20 minutes. The reaction mixture was stirred for 2 hours at room temperature . Another 1.5 ml glacial acetic acid was added dropwise and stirring was continued for 30 minutes at room temperature. Et₂O was added to the reaction mixture and the ether fraction was washed 2 times with 1 N NaOH. The combined organic fractions were dried (Na₂SO₄). The drying agent was removed by filtration and the solvent by evaporation under reduced pressure. The residue was chromatographed (SiO₂) using CH₂Cl₂/CH₃OH/NH₃ 92/7.5/0.5 as the eluent to give 1.80 g (50.4%) of the N-methylated alcohol **Q16**-OH as a thick oil.

### Scheme 12-17 step iii:

PPh₃ (3.1 g, 11.9 mmol) and imidazole (0.83 g, 11.9 mmol) were dissolved in 100 ml CH₂Cl₂. I₂ (3.1 g, 11.9 mmol) was added and the resulting suspension was stirred for 20 minutes at room temperature . A solution of the alcohol **Q16**-OH (from step ii)(1.8 g, 9.47 mmol) in 8 ml CH₂Cl₂ was added dropwise and the reaction mixture was stirred for 20 hours at room temperatu re. H₂O was added and after separation the CH₂Cl₂ fraction was dried (Na₂SO₄). The drying agent was removed by filtration and the solvent by evaporation under reduced pressure. The residue was chromatographed (SiO₂) using CH₂Cl₂ as eluent to give 2.18 g (76.8%) of the iodide **Q16-I** as a thick oil.

**Q14** needed another approach:

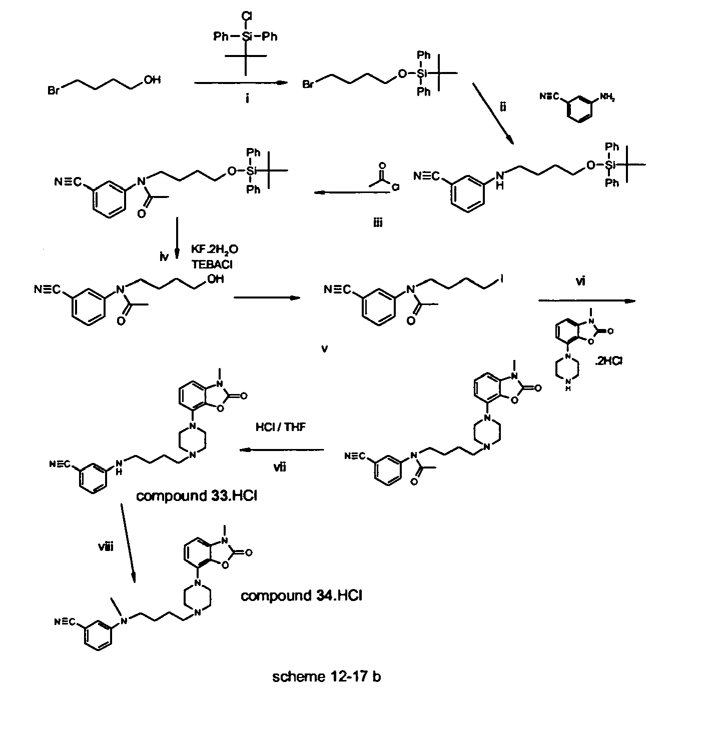

### Scheme 12-17 b step i:

To a stirred solution of 4-bromo-1-butanol (12 g, 78.4 mmol), imidazole (5.86 g, 86.2 mmol) in 70 ml DMF was added tert-butyldiphenylsilylchloride (20.4 ml, 78.4 mmol). The reaction mixture was stirred for 72 hours at room temperature The DMF was removed by evaporation under reduced pressure after which H₂O and CH₂Cl₂ were added. The CH₂Cl₂ fraction was dried (Na₂SO₄). The drying agent was removed by filtration and the solvent by evaporation under reduced pressure. The residue was chromatographed (SiO₂) using CH₂Cl₂/petroleum ether 1:4 as eluent to give 16.6 g (54.2%) of the silylated alcohol as an oil.

### Scheme 12-17 b step ii:

A mixture of 3-cyanoaniline (4.53 g, 38.36 mmol), silylated alcohol (from step i)(15 g, 38.36 mmol), DIPEA (6.64 ml, 38.36 mmol) and Nal (11.5 g, 76.73 mmol) in 400 ml butyronitrile was refluxed for 24 h. The solvent was evaporated under reduced pressure. H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂ as eluent to give 7.17 g (43.7%) of the alkylated aniline as an oil.

### Scheme 12-17 b step iii:

A mixture of the alkylated aniline (from step ii)(1 g, 2.34 mmol), acetyl chloride (0.17 ml, 2.34 mmol), DIPEA (0.40 ml, 2.34 mmol) in 15 ml CH₂Cl₂ was stirred at room temperature for 1 h. The solvent was evaporated under reduced pressure and the residue was chromatographed (SiO₂) using CH₂Cl₂/MeOH 98:2 as the eluent to give 0.88 g (80%) of the acylated aniline as a thick oil.

### Scheme 12-17 b step iv:

A mixture of the acylated aniline (from step iii)(0.88 g, 1.87 mmol), KF.2H₂O (0.26 g, 2.81 mmol), benzyltriethylammoniumchloride (0.37 g, 2.06 mmol) in 21 ml CH ₃CN was refluxed for 4 h. The solvent was evaporated under reduced pressure. H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH/NH₃ 92/7.5/0.5 as the eluent to give 0.31 g (71.4%) of the alcohol as a thick oil.

### Scheme 12-17 b step v:

The conversion of the resulting alcohols to the corresponding iodo derivatives was executed according to the procedure described in scheme 12-17 step iii.

### Scheme 12-17 b step vi:

A mixture of the piperazine (0.41 g, 1.34 mmol), the iodide from step v (0.46 g, 1.34 mmol), DIPEA (0.88 ml, 5.11 mmol) and Nal (0.2 g, 1.34 mmol) in 50 ml CH ₃CN was refluxed for 24 h. The solvent was evaporated under reduced pressure. H₂O and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH/NH₃ 96/3.75/0.25 as eluent to give 0.25 g (41.7%) of the alkylated piperazine as a thick oil.

### Scheme 12-17 b step vii:

To a stirred solution of the alkylated piperazine from step vi (0.25 g, 0.56 mmol) in 10 ml THF was added 10 ml 1N HCl. The reaction mixture was reluxed for 6 h. Nothing had happened, so 5 ml of concentrated HCl (36-38%) was added and the reaction mixture was refluxed for 24 h. The THF and H₂O were removed by evaporation under reduced pressure after which a K₂CO₃ solution and CH₂Cl₂ were added. The CH₂Cl₂ was dried (Na₂SO₄) and evaporated under reduced pressure. The residue was chromatographed (SiO₂) using CH₂Cl₂/CH₃OH/NH₃ 96/3.75/0.25 as eluent. The residual oil was dissolved in ethtylacetate and 1 eq. of AcCI/EtOH (1.0 M) was added. The precipitate was filtered to give 0.25 g (100%) of the de -acylated product as a white solid, containing compound **33**.HCl, m.p. 174-176 °C.

### Scheme 12-17 b step viii:

Compound **34**.HCl was made from **33**.HCl by a simple methylation according to the procedure described in scheme VI, step ii. Formation of the HCl salt by treatment of 1 equivalent 1 M AcCl/MeOH.

### Synthesis of Q20-21:

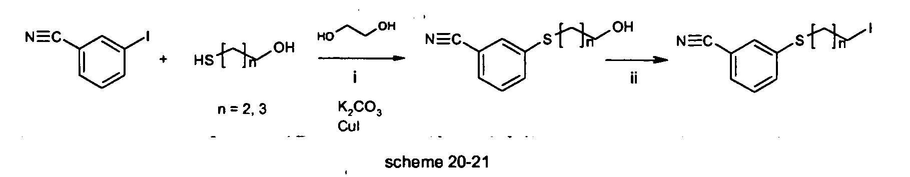

### Scheme 20-21 step i:

The starting iodobenzene and alcohols were commercially available.

A mixture of 3-iodobenzonitrile (2.29 g, 10 mmol), 4-mercapto-1-butanol (1.03 ml, 10 mmol), ethylene glycol (1.12 ml, 20 mmol), K₂CO₃ (2.78 g, 20 mmol) and Cul (95 mg, 0.5 mmol) in 40 ml 2-propanol under a nitrogen atmosphere was refluxed for 24 h. The solvent was evaporated under reduced pressure. Aqueous NH₄OH and CH₂Cl₂ were added to the residue and after separation the organic layer was dried (Na₂SO₄). The drying agent was removed by filtration and the solvent by evaporation under reduced pressure. The residue was chromatographed (SiO₂) using CH₂Cl₂/CH₃OH/NH₃ 96:3.75:0.25 as the eluent to give 1.40 g (67.6%) of the thioalkylated benzene **Q21**-OH as an oil.

### Scheme 20-21 step ii:

The conversion of the resulting alcohols to the corresponding iodo derivatives was performed according to the procedure described in scheme 12-17 step iii.

**Q20**-OH was prepared analogously to **Q21**-OH.

### Synthesis of Q22-23:

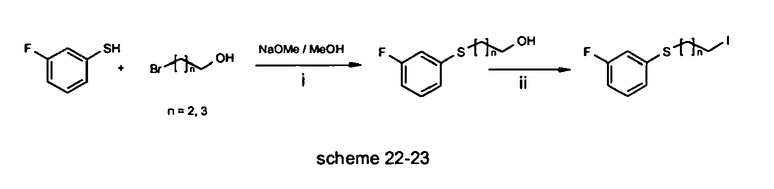

The starting phenol and alcohols were commercially available.

### Scheme 22-23 step i:

To a stirred solution of NaOMe (8.1 g, 150 mmol) in 100 ml methanol under a nitrogen atmosphere, was added dropwise 3-fluorothiophenol (9.5 ml, 100 mmol). The reaction mixture was stirred for 10 minutes at room temperature . 3-bromo-1-propanol (13.6 ml, 150 mmol) was added in one time, after which the reaction mixture was refluxed for 14 hours. After the reaction was allowed to cool, the mixture was concentrated in vacuo after which H₂O and CH₂Cl₂ were added to the residue and after separation the organic layer was dried (Na ₂SO₄). The drying agent was removed by filtration and the solvent by evaporation under reduced pressure. The residue was chromatographed (SiO₂) using CH₂Cl₂/CH₃OH 99/1 as eluent to give 18.5 g (99.5%) of the alkylated product as an oil containing **Q22**-OH.

### Scheme 22-23 step ii:

The conversion of the resulting alcohols to the corresponding iodo derivatives was executed according to the procedure described in scheme 12-17 step iii.

**Q23**-OH was prepared analogously to **Q22**-OH.

The specific compounds of which the synthesis is described above are intended to further illustrate the invention in more detail, and therefore are not deemed to restrict the scope of the invention in any way. Other embodiments of the invention will be apparent to those skilled in the art from consideration of the specification and practice of the invention disclosed herein. It is thus intended that the specification and examples be considered as exemplary only, with a true scope and spirit of the invention being indicated by the claims.

### ABBREVIATIONS

AcCl acetylchloride
ADDP 1,1'-(azodicarbonyl)dipiperidine
CDI carbonyldiimidazol
Dba see Huang et al., J. Am.Chem.Soc., 125(2003)6653
DCE dichloroethane
DCM dichloromethane
DIAD diisopropyldiazodicarboxylate
DIPE diisopropylether
DIPEA diisopropylethylamine

| | | | |
|---|---|---|---|
| CH₂Cl₂(ml) | MeOH(ml) | NH₄OH(ml) | |
| DMA 0.125 | 980 | 18.75 | 1.25 |
| DMA 0.187 | 970 | 28.13 | 1.87 |
| DMA 0.25 | 960 | 37.5 | 2.5 |
| DMA 0.50 | 920 | 75.0 | 5.0 |
| DMA 0.75 | 880 | 112.5 | 7.5 |
| DMA 1.00 | 840 | 150.0 | 10.0 |

DMAP 4-dimethylaminopyridin
DME dimethoxyethane
DMF N,N-dimethylformamide
EtOH ethanol
MeOH methanol
MTBE methyl(*tert.*)-butylether
NMP N-methylpyrrolidon
PA petroleum ether
TBAB tetrabutylammoniumbromide
TBAC tetrabutylammoniumchloride
TBAF tetrabutylammoniumfluoride
THF tetrahydrofurane
XPHOS see Huang et al., J. Am.Chem.Soc., 125(2003)6653

### EXAMPLE: FORMULATION OF COMPOUND 14 USED IN ANIMAL STUDIES

**For oral *(p.o.)* administration :** to the desired quantity (0.5-5 mg) of the solid compound 14 in a glass tube, some glass beads were added and the solid was milled by vortexing for 2 minutes. After addition of 1 ml of a solution of 1% methylcellulose in water and 2% (v/v) of Poloxamer 188 (Lutrol F68), the compound was suspended by vortexing for 10 minutes. The pH was adjusted to 7 with a few drops of aqueous NaOH (0.1N). Remaining particles in the suspension were further suspended by using an ultrasonic bath.

**For intraperitoneal *(i.p.)* administration:** to the desired quantity (0.5-15 mg) of the solid compound 14 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE: PHARMACOLOGICAL TESTRESULTS

**Table 2. In vitro affinities and functional activity of compounds of the invention**

| | | | |
|---|---|---|---|
| Dopamine-D₂ and serotonin reuptake receptor affinity data obtained according to the protocols given above are shown in the table below. *In vitro* functional activity at cloned human dopamine D_{2,L} receptors as measured by accumulation of radiolabeled cAMP (intrinsic activity ε). In the lower part of the table, for comparison in vitro receptor binding data are given of benzoxazolone derivatives bearing a terminal phenyl group as disclosed in EP 0 900 792 It concerns the examples A1, A6, A12, A17, A18 and A22 as given in Table A on p. 14 of EP 0 900 792 A1. From the results it is clear that these compounds have a high affinity for dopamine -D₂ receptors, but are essentially devoid of serotonin reuptake inhibition. | | | |

| | **Dopamine-D₂** | **5-HT reuptake** | **Dopamine-D₂** |
|---|---|---|---|
| | | | |
| | **binding** | **binding** | **cAMP accum** |
| | | | |
| **compound** | **pK₁** | **pK₁** | ε (intrinsic activity) |
| | | | |
| **6** | **9.0** | **8.4** | **0.76** |
| **9** | **8.2** | **7.0** | **0.64** |
| **14** | **7.8** | **9.0** | **0.57** |
| **15** | **8.2** | **9.1** | **0.61** |
| **17** | **7.6** | **7.3** | **0.51** |
| **20** | **8.4** | **8.5** | **0.98** |
| **24** | **8.9** | **7.9** | **0.86** |
| **26** | **8.0** | **8.0** | **0.79** |
| **31** | **7.2** | **8.3** | **0.56** |
| **35** | **7.0** | **9.2** | **0.55** |
| | | | |
| **Examples from Table A on page 14 of** EP 0 900 792 A1 | | | |
| | | | |
| **A1** | **8.9** | **6.1** | **-** |
| **A6** | **8.3** | **< 6.3** | **-** |
| **A12** | **7.9** | **< 6.3** | **-** |
| **A17** | **7.6** | **6.3** | **-** |
| **A18** | **9.2** | **5.9** | **-** |
| **A22** | **8.3** | **< 6.3** | **-** |

## Claims

1. Compounds of the general formula (1): 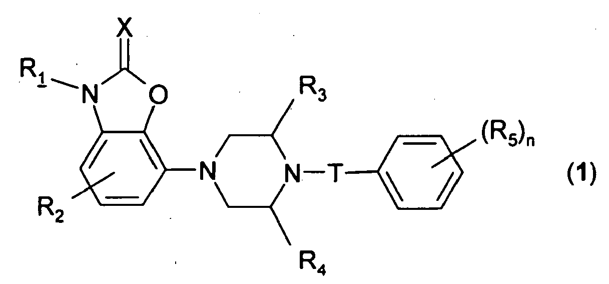 wherein: X = S or O,
R₁ is H, (C₁-C₆)alkyl, CF₃, CH₂CF₃, OH or O-(C₁-C₆)alkyl
R₂ is H, (C₁-C₆)alkyl, halogen or cyano
R₃ is H or (C₁-C₆)alkyl
R₄ is H, (C₁-C₆)alkyl, optionally substituted with a halogen atom,
wherein: 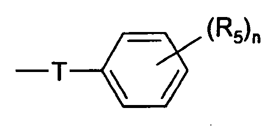 is selected from the group consisting of: 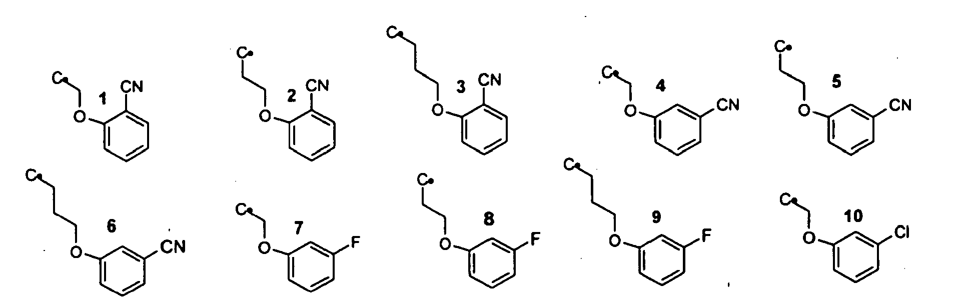 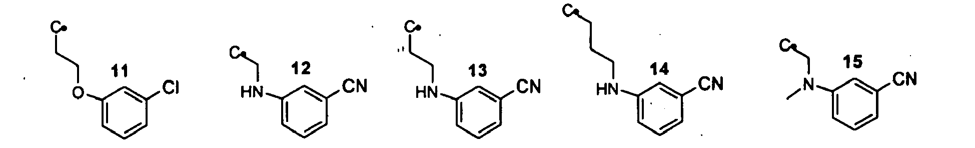 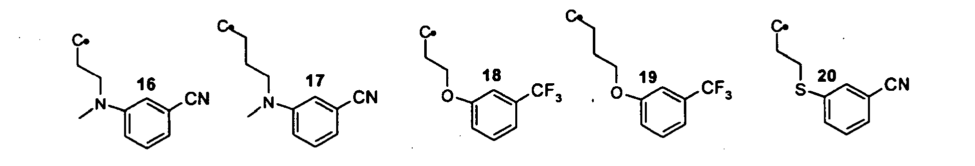 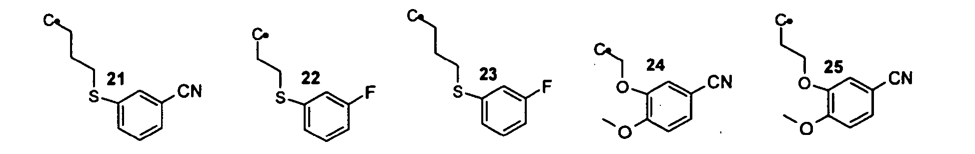 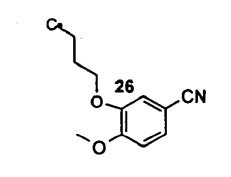 and tautomers, stereoisomers and N-oxides thereof, as well as pharmacologically acceptable salts, hydrates and solvates of said compounds of formula (1) and its tautomers, stereoisomers and N-oxides.

2. Compounds of the formula (1) as claimed in claim 1, wherein the phenylpiperazine part of the molecule is selected from the group consisting of: 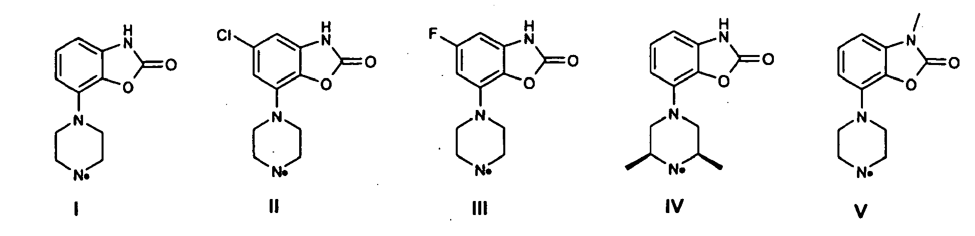 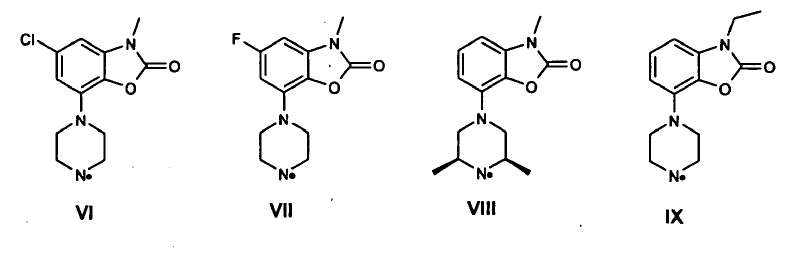 and wherein the second part of the molecule is selected from the group consisting of: 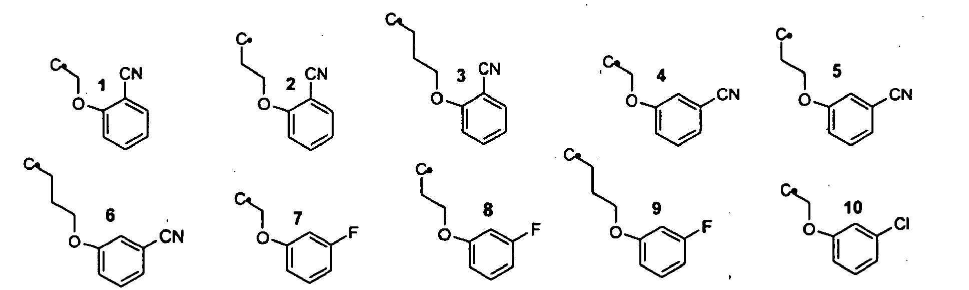 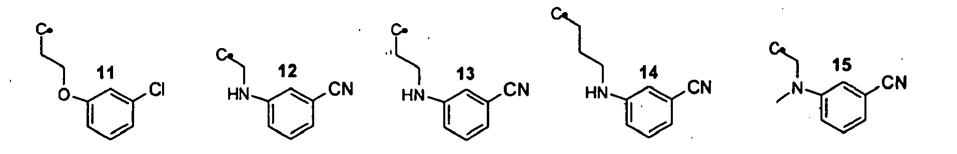 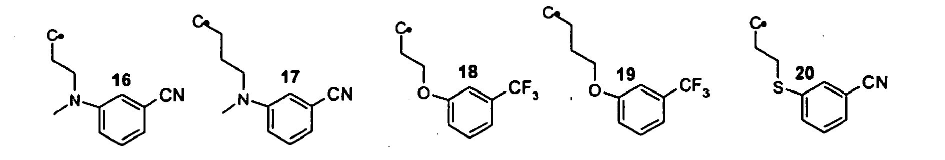 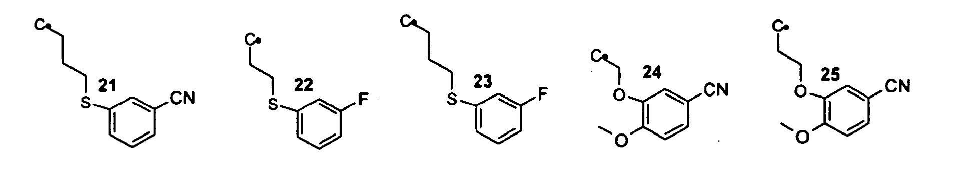 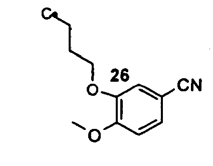 and tautomers, stereoisomers and N-oxides thereof, as well as pharmacologically acceptable salts, hydrates and solvates of said compounds of formula (1) and its tautomers, stereoisomers and N-oxides.

3. A pharmaceutical composition comprising, in addition to a pharmaceutically acceptable carrier and/or at least one pharmaceutically acceptable auxiliary substance, a pharmacologically active amount of at least one compound of claim 1 or claim 2, or a salt thereof, as an active ingredient.

4. A method of preparing a composition as claimed in claim 3, **characterised in that** at least one compound of claim 1 or claim 2, or a salt thereof, is brought into a form suitable for administration

5. A compound as claimed in claim 1 or claim 2, or a salt thereof, for use as a medicament.

6. Use of a compound as claimed in claim 1 or claim 2 for the preparation of a pharmaceutical composition for the treatment of CNS disorders.

7. Use as claimed in claim 6, **characterized in that** said disorders are aggression, anxiety disorders, autism, vertigo, depression, disturbances of cognition or memory, Parkinson's disease, schizophrenia and other psychotic disorders.

8. Use as claimed in claim 6, **characterized in that** said disorder is depression.

9. Use as claimed in claim 6, **characterized in that** said disorders are schizo-phrenia and other psychotic disorders.

10. Use as claimed in claim 6, **characterized in that** said disorder is Parkinson's disease.

## Patentansprüche

1. Verbindungen der allgemeinen Formel (1): 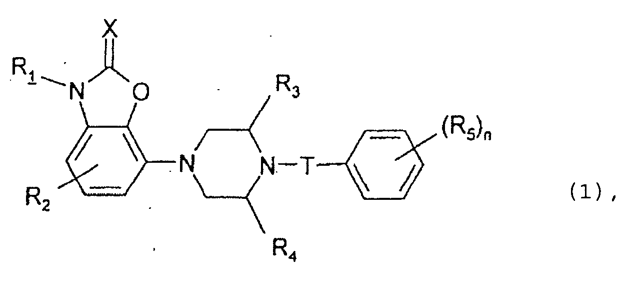 worin: X = S oder O,
R₁ für H, (C₁-C₆)-Alkyl, CF₃, CH₂CF₃, OH oder O-(C₁-C₆)-Alkyl steht,
R₂ für H, (C₁-C₆)-Alkyl, Halogen oder Cyano steht,
R₃ für H oder (C₁-C₆)-Alkyl steht,
R₄ für H, (C₁-C₆)-Alkyl steht, gegebenenfalls substituiert mit einem Halogenatom,
worin: 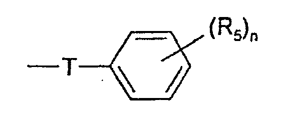 ausgewählt wird aus der Gruppe bestehend aus: 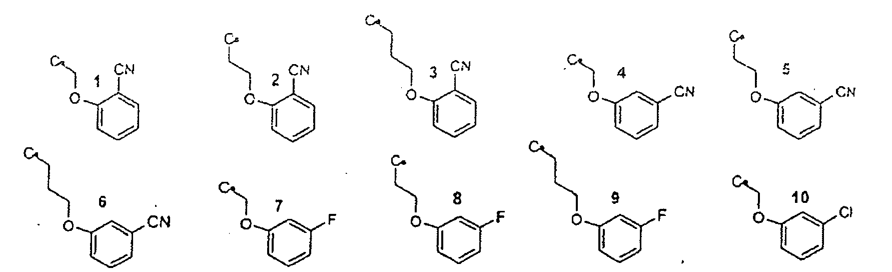 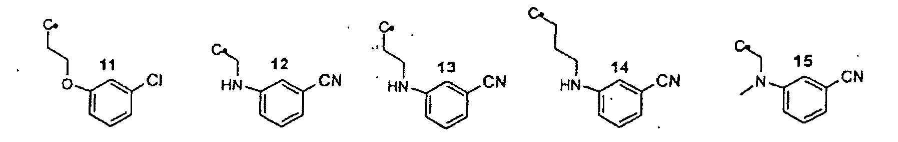 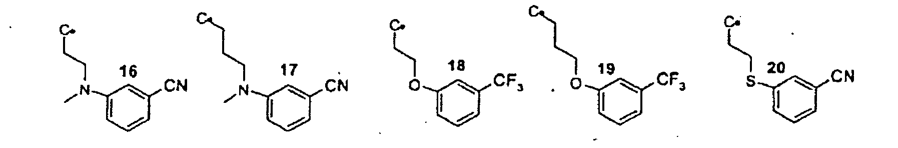 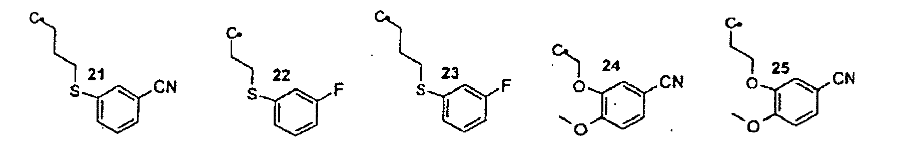 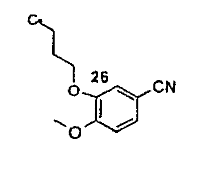 und Tautomere, Stereoisomere und N-Oxide davon, als auch pharmakologisch annehmbare Salze, Hydrate und Solvate von besagten Verbindungen der Formel (1) und ihren Tautomeren, Stereoisomeren und N-Oxiden.

2. Verbindungen der Formel (1) wie in Anspruch 1 beansprucht, worin der Phenylpiperazinteil des Moleküls ausgewählt wird aus der Gruppe bestehend aus: 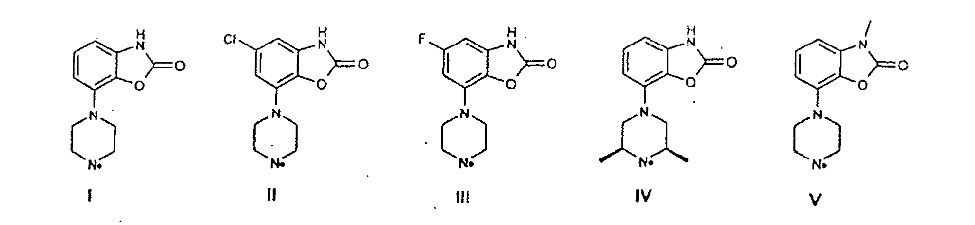 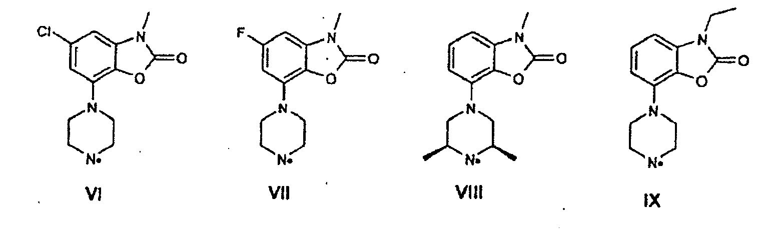 und worin der zweite Teil des Moleküls ausgewählt wird aus der Gruppe bestehend aus: 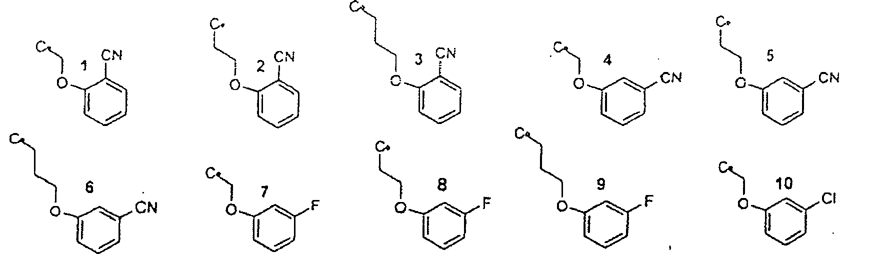 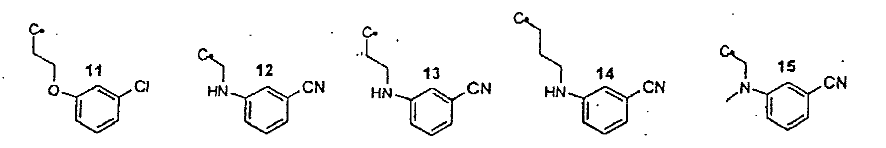 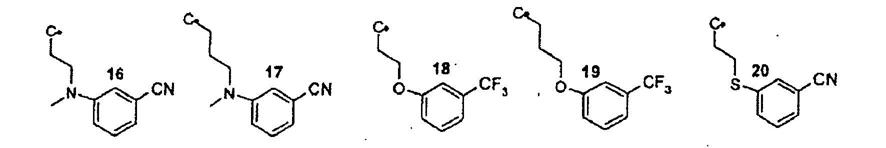 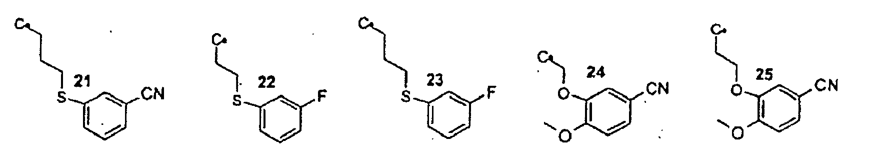 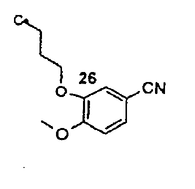 und Tautomere, Stereoisomere und N-Oxide davon, als auch pharmakologisch annehmbare Salze, Hydrate und Solvate von besagten Verbindungen der Formel (1) und ihren Tautomeren, Stereoisomeren und N-Oxiden.

3. Pharmazeutische Zusammensetzung, welche zusätzlich zu einem pharmazeutisch annehmbaren Träger und/oder mindestens einer pharmazeutisch annehmbaren Hilfssubstanz eine pharmakologisch wirksame Menge von mindestens einer Verbindung nach Anspruch 1 oder Anspruch 2 oder ein Salz davon als einen wirksamen Inhaltsstoff umfasst.

4. Verfahren zum Herstellen einer Zusammensetzung wie in Anspruch 3 beansprucht, **dadurch gekennzeichnet, dass** mindestens eine Verbindung nach Anspruch 1 oder Anspruch 2 oder ein Salz davon in eine für Verabreichung geeignete Form gebracht wird.

5. Verbindung wie in Anspruch 1 oder Anspruch 2 beansprucht oder ein Salz davon zur Verwendung als ein Medikament.

6. Verwendung einer Verbindung wie in Anspruch 1 oder Anspruch 2 beansprucht für die Herstellung einer pharmazeutischen Zusammensetzung für die Behandlung von CNS-Störungen.

7. Verwendung wie in Anspruch 6 beansprucht, **dadurch gekennzeichnet, dass** besagte Störungen Aggression, Angststörungen, Autismus, Schwindel, Depression, Störungen von Kognition oder Gedächtnis, Parkinson-Krankheit, Schizophrenie und andere psychotische Störungen sind.

8. Verwendung wie in Anspruch 6 beansprucht, **dadurch gekennzeichnet, dass** besagte Störung Depression ist.

9. Verwendung wie in Anspruch 6 beansprucht, **dadurch gekennzeichnet, dass** besagte Störungen Schizophrenie und andere psychotische Störungen sind.

10. Verwendung wie in Anspruch 6 beansprucht, **dadurch gekennzeichnet, dass** besagte Störung Parkinson-Krankheit ist.

## Revendications

1. Composés de formule générale (1) : 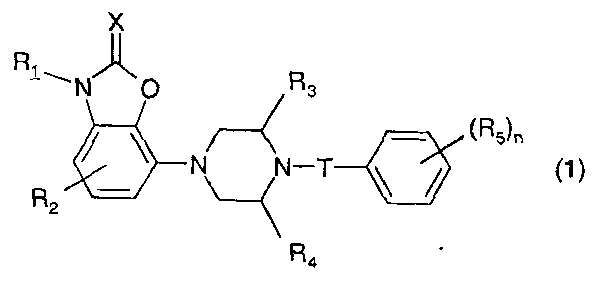 où : X = S ou O ;
R₁ est H, (C₁-C₆)alkyle, CF₃, CH₂CF₃, OH ou O-(C₁-C₆)alkyle
R₂ est H, (C₁-C₆)alkyle, halogène ou cyano
R₃ est H ou (C₁-C₆)alkyle
R₄ est H, (C₁-C₆)alkyle, éventuellement substitué avec un atome d'halogène,
où : 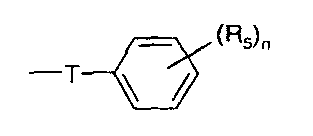 est choisi dans le groupe consistant en : 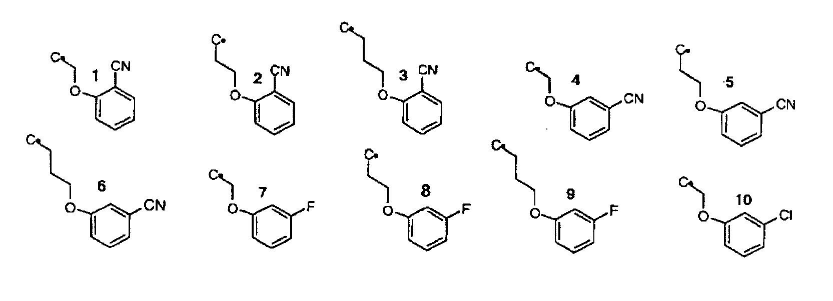 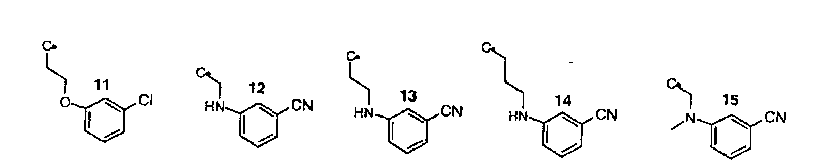 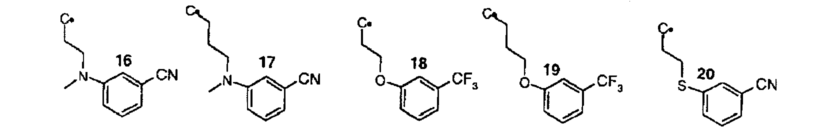 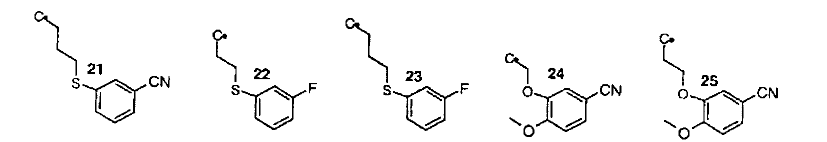 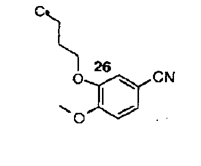 et leurs tautomères, stéréoisomères et N-oxydes, ainsi que les sels, hydrates et solvates pharmacologiquement acceptables desdits composés de formule (1) et de leurs tautomères, stéréoisomères et N-oxydes.

2. Composés de formule (1) selon la revendication 1 où la partie phénylpipérazine de la molécule est choisie dans le groupe consistant en : 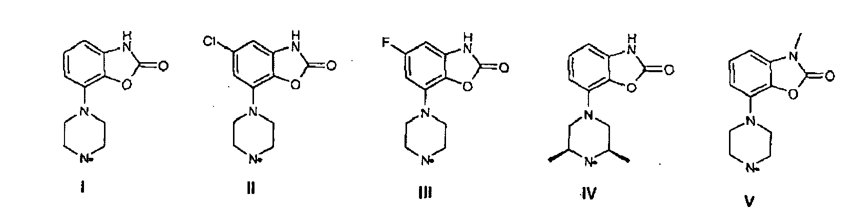 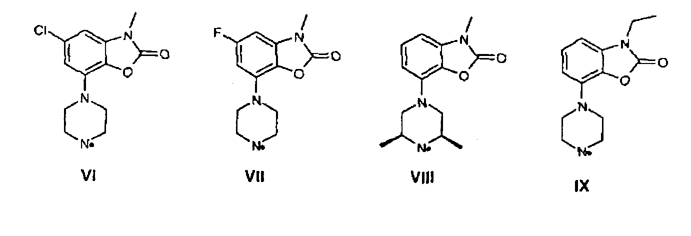 et où la seconde partie de la molécule est choisie dans le groupe consistant en : 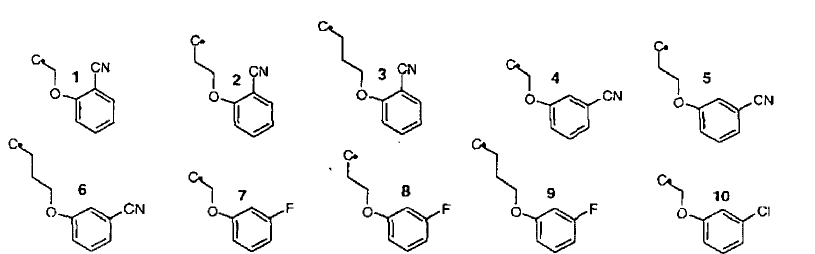 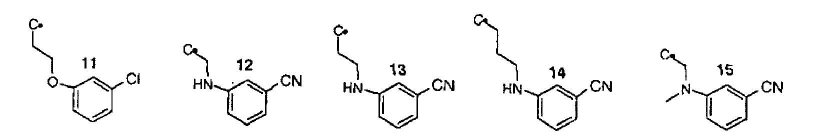 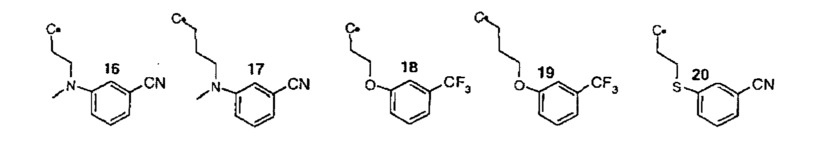 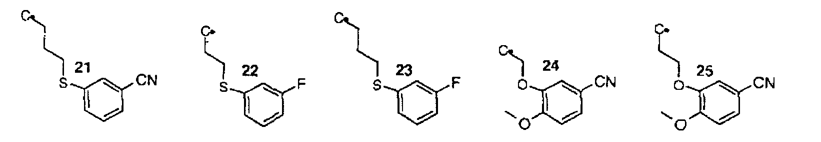 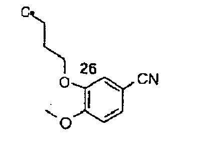 et leurs tautomères, stéréoisomères et N-oxydes, ainsi que les sels, hydrates et solvates pharmacologiquement acceptables desdits composés de formule (1) et de leurs tautomères, stéréoisomères et N-oxydes.

3. Composition pharmaceutique comprenant, en plus d'un vecteur pharmaceutiquement acceptable et/ou d'au moins une substance auxiliaire pharmaceutiquement acceptable, une quantité pharmacologiquement active d'au moins un composé selon la revendication 1 ou la revendication 2, ou d'un sel de celui-ci, comme ingrédient actif.

4. Procédé de préparation d'une composition selon la revendication 3, **caractérisé en ce qu'**au moins un composé selon la revendication 1 ou la revendication 2, ou un sel de celui-ci, est mis sous une forme appropriée pour l'administration.

5. Composé selon la revendication 1 ou la revendication 2, ou sel de celui-ci, destiné à être utilisé comme médicament.

6. Utilisation d'un composé selon la revendication 1 ou la revendication 2 pour la préparation d'une composition pharmaceutique pour le traitement des troubles du SNC.

7. Utilisation selon la revendication 6 **caractérisée en ce que** lesdits troubles sont l'agression, les troubles de type anxiété, l'autisme, le vertige, la dépression, les perturbations de la cognition ou de la mémoire, la maladie de Parkinson, la schizophrénie et d'autres troubles psychotiques.

8. Utilisation selon la revendication 6 **caractérisée en ce que** ledit trouble est de la dépression.

9. Utilisation selon la revendication 6 **caractérisée en ce que** lesdits troubles sont la schizophrénie et d'autres troubles psychotiques.

10. Utilisation selon la revendication 6 **caractérisée en ce que** ledit trouble est la maladie de Parkinson.
